# EUROPÄISCHE PATENTSCHRIFT

(11) **EP 3 501 464 B1**
(45) Veröffentlichungstag und Bekanntmachung des Hinweises auf die Patenterteilung: **06.04.2022**
(21) Anmeldenummer: 17209124.1
(22) Anmeldetag: 20.12.2017
(51) Int. Cl.: A61F 9/06

(54) **SCHUTZKASSETTE FÜR EINE BLENDSCHUTZVORRICHTUNG**
PROTECTIVE CARTRIDGE FOR AN ANTIGLARE DEVICE
CASSETTE DE PROTECTION POUR UN DISPOSITIF ANTI-REFLET

(43) Veröffentlichungstag der Anmeldung: 26.06.2019
(73) Patentinhaber: Optrel Holding AG, 9630 Wattwil (CH)
(72) Erfinder: BISCHOF, David, 6832 Sulz (AT); ESPOSITO, Martin, 8640 Rapperswil-Jona (CH); MICHLER, Markus, 6800 Feldkirch (AT)
(74) Vertreter: Daub, Thomas

(56) Entgegenhaltungen:
- WO-A1-2004/053586
- WO-A1-2015/034575
- WO-A1-2017/196721
- US-A1- 2014 168 546
- US-B2- 9 164 314

## Beschreibung

Die Erfindung betrifft eine Schutzkassette für eine Blendschutzvorrichtung nach dem Oberbegriff des Anspruchs 1, sowie eine Blendschutzvorrichtung nach Anspruch 9.

Es ist bereits eine Schutzkassette für eine Blendschutzvorrichtung, mit einem optischen Blendschutzfilter, mit zumindest einer Steuer- und/oder Regeleinheit, welche dazu vorgesehen ist, abhängig von einem erfassten Arbeitszustand, einem elektronischen Signal einer externen Signalquelle und/oder einer Lichteinstrahlung eine Durchlässigkeit des optischen Blendschutzfilters zumindest zwischen zumindest einer Hellstufe und zumindest einer Dunkelstufe zu steuern und/oder zu regeln, vorgeschlagen worden. Entsprechende optische Blendschutzfilter weisen in der Hellstufe eine niedrige Durchlässigkeit auf, welche bedingt, dass ein Benutzer der Blendschutzvorrichtung zu einer sicheren Orientierung im Raum und/oder zu einer präzisen Werkstückkontrolle eines durch den Benutzer bearbeiteten Werkstücks die Blendschutzvorrichtung aus seinem Sichtfeld entfernen muss, was zu einer Einschränkung einer Arbeitssicherheit und/oder Einschränkung einer Arbeitsgeschwindigkeit führen kann.

Beispielsweise offenbaren die Druckschriften WO2017/196721 A1, US2014/168546A1 und WO2015/034575A1 jeweils eine Schutzkassette für eine Blendschutzvorrichtung, mit einem optischen Blendschutzfilter, mit zumindest einer Steuer- und/oder Regeleinheit, welche dazu vorgesehen ist, abhängig von einem erfassten Arbeitszustand, einem elektronischen Signal einer externen Signalquelle und/oder einer Lichteinstrahlung eine Durchlässigkeit des optischen Blendschutzfilters zumindest zwischen zumindest einer Hellstufe und zumindest einer Dunkelstufe zu steuern und/oder zu regeln, wobei die Durchlässigkeit des optischen Blendschutzfilters in zumindest einer Hellstufe einer Schutzstufe kleiner als 2,5 entspricht.

Die Aufgabe der Erfindung besteht insbesondere darin, eine gattungsgemäße Vorrichtung mit verbesserten Eigenschaften hinsichtlich einer Durchlässigkeit bereitzustellen. Die Aufgabe wird erfindungsgemäß durch die Merkmale der Patentansprüche 1 und 9 gelöst, während vorteilhafte Ausgestaltungen und Weiterbildungen der Erfindung den Unteransprüchen entnommen werden können.

### Vorteile der Erfindung

Die Erfindung geht aus von einer Schutzkassette für eine Blendschutzvorrichtung, mit einem optischen Blendschutzfilter, welcher zumindest eine Flüssigkristallzelle aufweist, mit zumindest einer Steuer- und/oder Regeleinheit, welche dazu vorgesehen ist, abhängig von einem erfassten Arbeitszustand, einem elektronischen Signal einer externen Signalquelle und/oder einer Lichteinstrahlung eine Durchlässigkeit des optischen Blendschutzfilters zumindest zwischen zumindest einer Hellstufe und zumindest einer Dunkelstufe zu steuern und/oder zu regeln.

Es wird vorgeschlagen, dass die Durchlässigkeit des optischen Blendschutzfilters in zumindest einer Hellstufe einer Schutzstufe kleiner als 2,5, vorzugsweise höchstens 2,4, vorteilhaft höchstens 2,25, besonders vorteilhaft höchstens 2, bevorzugt höchstens 1,8 und besonders bevorzugt zumindest 1,7 entspricht. Dadurch können vorteilhaft verbesserte Eigenschaften hinsichtlich einer Durchlässigkeit erreicht werden, insbesondere in zumindest einer Hellstufe des optischen Blendschutzfilters. Zudem kann vorteilhaft eine hohe Flexibilität erreicht werden, insbesondere indem ein großer Bereich an Schutzstufen abgedeckt werden kann. Außerdem kann vorteilhaft ein hoher Benutzerkomfort erreicht werden, insbesondere indem einem Benutzer in der Hellstufe eine für eine Orientierung im Raum ausreichende Sicht durch den optischen Blendschutzfilter bereitgestellt werden kann. Vorteilhaft kann eine Sicherheit erhöht werden, insbesondere indem eine Notwendigkeit eines temporären Entfernens des optischen Blendschutzfilters aus einem Sichtfeld eines Benutzers zu einer Orientierung vermieden werden kann und/oder indem eine gefahrlose Orientierung im Raum mit dem optischen Blendschutzfilter in dem Sichtfeld des Benutzers ermöglicht werden kann. Zudem kann, beispielsweise bei einem Einsatz des optischen Blendschutzfilters zu Schweißarbeiten, vorteilhaft eine Arbeitserleichterung für einen Benutzer erreicht werden, insbesondere indem ein Ergebnis eines, insbesondere Licht emittierenden, Arbeitsschrittes direkt im Anschluss an die Lichtemission, insbesondere ohne ein Entfernen des optischen Blendschutzfilters aus dem Blickfeld des Benutzers, kontrolliert werden kann. Dadurch kann vorteilhaft eine hohe Arbeitsgeschwindigkeit erreicht werden. Insbesondere erfüllt die Blendschutzvorrichtung mit der Schutzkassette die Anforderungen der Normen ISO 16321-1 und ISO 16321-2.

Vorzugsweise ist die Schutzkassette als eine Blendschutzkassette, insbesondere eine Schweißhelmschutzkassette, ausgebildet, welche insbesondere in einer tragbaren, insbesondere zumindest auf einen Kopf eines Benutzers aufsetzbaren, Blendschutzvorrichtung angeordnet ist. Insbesondere ist die Schutzkassette im getragenen Zustand in einem Sichtfeld eines Benutzers angeordnet und/ oder gegenüber den Augen des Benutzers positionsfest angeordnet. Insbesondere ist die Schutzkassette relativ zu der Blendschutzvorrichtung derart angeordnet, dass außerhalb der Blendschutzvorrichtung, insbesondere innerhalb eines Sichtfelds des Benutzers, emittiertes Licht die Augen des Benutzers nur durch den optischen Blendschutzfilter gefiltert erreichen kann, insbesondere abgesehen von minimalem von außerhalb des Sichtfelds gestreutem Streulicht. Unter einer "Schutzkassette" soll in diesem Zusammenhang insbesondere ein vorzugsweise zusammenhängendes Modul der Blendschutzvorrichtung verstanden werden. Vorzugsweise weist die Schutzkassette insbesondere zumindest teilweise ein gemeinsames Gehäuse auf, in und/oder an welchem zumindest ein wesentlicher Teil der Bauteile der Schutzkassette angeordnet sind. Bevorzugt soll darunter insbesondere ein eigenständiges Modul verstanden werden, welches vorzugsweise selbstständig funktionsfähig ist. Grundsätzlich wäre jedoch auch denkbar, dass die Schutzkassette in einer weiteren Einheit der Blendschutzvorrichtung, wie beispielsweise einer Schildeinheit und/oder einem Schweißhelm, integriert ist. Unter der Wendung "in einer weiteren Einheit der Blendschutzvorrichtung integriert" soll insbesondere verstanden werden, dass die Schutzkassette zumindest teilweise einstückig mit der weiteren Einheit der Blendschutzvorrichtung ausgebildet ist und/oder zumindest im Wesentlichen unlösbar, insbesondere nicht zerstörungsfrei lösbar, mit der weiteren Einheit der Blendschutzvorrichtung, insbesondere zumindest einem Gehäuseteil der Schildeinheit und/oder des Schweißhelms und/oder zumindest einem Rahmenteil der Schildeinheit und/oder des Schweißhelms, verbunden ist und/oder einen festen Bestandteil der Blendschutzvorrichtung ausbildet. Unter "einstückig" soll insbesondere stoffschlüssig verbunden, wie beispielsweise durch einen Schweißprozess und/oder Klebeprozess usw., und besonders vorteilhaft angeformt, verstanden werden, wie durch die Herstellung aus einem Guss und/oder durch die Herstellung in einem Ein- oder Mehrkomponentenspritzverfahren. Unter "zumindest im Wesentlichen unlösbar" soll hier insbesondere eine Verbindung von zumindest zwei Elementen verstanden werden, die lediglich unter der Zuhilfenahme von Trennwerkzeugen, wie beispielsweise einer Säge, insbesondere einer mechanischen Säge usw., und/oder chemischen Trennmitteln, wie beispielsweise Lösungsmittel usw., voneinander trennbar sind.

Ferner soll in diesem Zusammenhang unter einer "Blendschutzvorrichtung" insbesondere eine Vorrichtung verstanden werden, welche zu einem Schutz eines Benutzers vor zu großer Helligkeit und/oder Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß-, Schneide-, Hartlöt- und/oder Schleifprozesses dient. Bevorzugt soll darunter insbesondere eine Vorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Schutzvorrichtung denkbar, wie beispielsweise als Schweißhelm, -schirm, -maske, -brille und/oder -schild. Unter einem "optischen Blendschutzfilter" soll insbesondere ein optischer Filter für eine Blendschutzvorrichtung verstanden werden, welcher insbesondere zumindest ein Schutzglas und/oder ein Kunststoffschutzglas umfasst. Der optische Blendschutzfilter ist insbesondere zumindest dazu vorgesehen, zumindest einen Teil eines auf den optischen Blendschutzfilter einfallenden Lichts zu absorbieren und/oder zu reflektieren. Insbesondere ist eine Absorption, insbesondere eine Stärke der Absorption, und/oder Reflexion, insbesondere eine Stärke der Reflexion, des optischen Blendschutzfilters wellenlängenabhängig. Beispielsweise ist denkbar, dass ein Teil des elektromagnetischen Spektrums, beispielsweise UV-Licht stärker absorbiert und/oder reflektiert wird als ein weiterer Teil des elektromagnetischen Spektrums, beispielsweise sichtbares Licht. Vorzugsweise absorbiert und/oder reflektiert der optische Blendschutzfilter UV-Licht nahezu vollständig, während insbesondere sichtbares Licht nur zu einem Teil, welcher insbesondere kleiner ist als 70 %, bevorzugt 60 %, von dem optischen Blendschutzfilter absorbiert und/oder reflektiert wird. Vorzugsweise ist eine Absorption, insbesondere eine Stärke der Absorption, und/oder Reflexion, insbesondere eine Stärke der Reflexion, des optischen Blendschutzfilters regelbar. Vorzugsweise soll unter dem optischen Blendschutzfilter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden.

Besonders bevorzugt weist der Blendschutzfilter zumindest eine in der Transmission schaltbare Flüssigkristallebene, insbesondere zumindest eine Flüssigkristallzelle, vorzugsweise zumindest zwei Flüssigkristallzellen, auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Des Weiteren soll unter einer "Steuer- und/oder Regeleinheit" in diesem Zusammenhang insbesondere eine Einheit mit zumindest einer Steuerelektronik verstanden werden. Unter einer "Steuerelektronik" soll insbesondere eine Einheit mit zumindest einer elektronischen Schaltung verstanden werden, welche vorzugsweise aus Spannungs- und Vergleichsregelbausteinen besteht. Grundsätzlich kann die Steuerelektronik jedoch auch komplexer aufgebaut sein, wie insbesondere durch die Nutzung einer anwendungsspezifischen integrierten Schaltung (ASIC) und/oder eines Mikrokontrollerbausteins. Unter einem "Arbeitszustand" soll insbesondere ein Betriebszustand zumindest eines Teils eines Arbeitsgeräts und/oder Werkzeugs verstanden werden. Vorzugsweise ist der Arbeitszustand als ein Betriebszustand eines Schweiß-, Hartlöt- und/oder Brennschneidgeräts wie beispielsweise eine Stromzufuhr, eine Brennstoffzufuhr und/oder eine Aktivierung des Schweiß-, Hartlöt- und/oder Brennschneidgeräts ausgebildet. Erfindungsgemäß weist die Blendschutzvorrichtung zumindest eine Informationsschnittstelle zu einem Empfang zumindest einer Information über zumindest einen Arbeitszustand und/oder zumindest einen Sensor zu einer Detektion zumindest eines Arbeitszustands auf. Insbesondere weist die Schutzkassette zumindest einen Lichtsensor, insbesondere einen Lichtintensitätssensor, auf, welcher insbesondere dazu vorgesehen ist, Messdaten zur Verarbeitung an die Steuer- und/oder Regeleinheit zu senden. Alternativ ist denkbar, dass die Schutzkassette zumindest Daten, beispielsweise elektronische Steuer und/oder Regelsignale, zumindest eines externen Lichtsensors, beispielsweise eines Lichtsensors der Blendschutzvorrichtung, und/oder einer weiteren externen Signalquelle empfängt und mittels der Steuer- und/oder Regeleinheit zu einer Steuerung und/oder Regelung der Durchlässigkeit des optischen Blendschutzfilters verarbeitet. Unter einer "Hellstufe" soll insbesondere eine Lichtdurchlässigkeitsstufe des optischen Blendschutzfilters mit maximaler Lichttransmission durch den optischen Blendschutzfilter verstanden werden. Vorzugsweise sind in der Hellstufe alle Flüssigkristallzellen des optischen Blendschutzfilters auf maximale Lichtdurchlässigkeit geschaltet. Unter einer "Dunkelstufe" soll insbesondere eine Lichtdurchlässigkeitsstufe des optischen Blendschutzfilters mit, insbesondere im Vergleich zu der Hellstufe, reduzierter Lichttransmission durch den optischen Blendschutzfilter verstanden werden. Vorzugsweise ist in der Dunkelstufe zumindest eine Flüssigkristallzelle des optischen Blendschutzfilters in einem, eine Lichtdurchlässigkeit reduzierenden und/oder beeinflussenden Zustand geschaltet. Vorzugsweise ist die Dunkelstufe, insbesondere die Reduzierung der Lichttransmission in der Dunkelstufe, stufenweise und/oder bevorzugt stufenlos regelbar. Insbesondere ist die Dunkelstufe, insbesondere die Reduzierung der Lichttransmission, durch den Benutzer einstellbar. Vorzugsweise ist die Dunkelstufe, insbesondere die Reduzierung der Lichttransmission, automatisch mittels der Steuer- und/oder Regeleinheit regelbar, insbesondere in Abhängigkeit einer gemessenen und/oder empfangenen Lichtintensität und/oder eines bestimmten Arbeitszustands.

Ferner sind verschiedene, einem Fachmann als sinnvoll erscheinende Flüssigkristallzellen denkbar, wie insbesondere eine TN-Flüssigkristallzelle mit der Twisted-Nematic-Technik. Insbesondere kann die TN Flüssigkristallzelle als "normally black" TN-Flüssigkristallzelle, vorzugsweise als "normally white" TN-Flüssigkristallzelle ausgebildet sein. Grundsätzlich wären jedoch auch andere, einem Fachmann als sinnvoll erscheinende Ausbildungen der Flüssigkristallzellen denkbar, wie beispielsweise als STN-Flüssigkristallzellen mit der Super-Twisted-Nematic-Technik, DSTN-Flüssigkristallzellen mit der Double-Super-Twisted-Nematic-Technik, TSTN-Flüssigkristallzellen mit der Triple-Super-Twisted-Nematic-Technik, VA-Flüssigkristallzellen mit der Vertical-Alignment-Technik, insbesondere PVA/MVA-Flüssigkristallzellen mit der Patterned-Vertical-Alignment-und/oder Multi-Domain-Vertical-Alignment-Technik, IPS-Flüssigkristallzellen mit der In-Plane-Switching-Technik, FLCD-Flüssigkristallzellen, also ferroelektrische Flüssigkristallzellen, und/oder TN-Flüssigkristallzellen mit der Guest-Host-Technik. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Unter einer "Schutzstufe" soll insbesondere eine Augenschutzstufe im Einklang mit den Normen DIN EN 166:2002-04 und/oder DIN EN 169:2003-02 verstanden werden. Insbesondere ist eine Transmission durch den optischen Blendschutzfilter von der Schutzstufe des optischen Blendschutzfilters abhängig. Insbesondere ist eine Transmission durch den optischen Blendschutzfilter umso größer umso kleiner die Schutzstufe ist.

Ferner wird vorgeschlagen, dass die Durchlässigkeit des optischen Blendschutzfilters in zumindest einer Dunkelstufe einer Schutzstufe von zumindest 3, vorzugsweise zumindest 5, vorteilhaft zumindest 10, besonders vorteilhaft zumindest 12, bevorzugt zumindest 15 und besonders bevorzugt höchstens 16 entspricht. Dadurch kann vorteilhaft eine hohe Sicherheit erreicht werden, insbesondere indem augengefährdendes Licht effektiv von den Augen des Benutzers ferngehalten werden kann. Zudem kann vorteilhaft eine hohe Flexibilität erreicht werden, insbesondere indem eine große Bandbreite an Schutzstufen abgedeckt werden kann.

Zudem wird vorgeschlagen, dass der optische Blendschutzfilter in zumindest einer Hellstufe in zumindest einem Spektralbereich eine Transmissivität von zumindest 29 %, vorzugsweise zumindest 33 %, bevorzugt zumindest 40 % und besonders bevorzugt höchstens 80 % aufweist. Dadurch können vorteilhaft verbesserte Eigenschaften hinsichtlich einer Durchlässigkeit von Licht erreicht werden, insbesondere in zumindest einer Hellstufe des optischen Blendschutzfilters. Außerdem kann vorteilhaft ein hoher Benutzerkomfort erreicht werden, insbesondere indem einem Benutzer in der Hellstufe eine für eine Orientierung im Raum ausreichende Sicht durch den optischen Blendschutzfilter bereitgestellt werden kann, wodurch vorteilhaft eine hohe Sicherheit erreicht werden kann. Unter einem "Spektralbereich" soll insbesondere zumindest ein Teilbereich des elektromagnetischen Spektrums verstanden werden, beispielsweise ein Infrarotbereich, ein Bereich sichtbaren Lichts, ein UV-Bereich und/oder eine zumindest teilweise Kombination des Infrarotbereichs, des Bereichs sichtbaren Lichts und/oder des UV-Bereichs. Vorzugsweise ist der Spektralbereich, welcher insbesondere die größte Transmissivität durch den optischen Blendschutzfilter aufweist, als zumindest ein, insbesondere in Richtung des Infrarotbereichs und/oder des UV-Bereichs abgegrenzter, Bereich des sichtbaren Lichts ausgebildet. Vorteilhaft umfasst der Spektralbereich zumindest Licht in einem Wellenlängenbereich welcher oberhalb 380 nm, vorzugsweise oberhalb 420 nm und bevorzugt oberhalb 470 nm, sowie unterhalb 550 nm, bevorzugt unterhalb 590 nm und besonders bevorzugt unterhalb 780 nm liegt. Insbesondere ist der Wert der Transmissivität als ein durchschnittlicher Transmissionswert von Licht des Spektralbereichs durch den optischen Blendschutzfilter bei einer Mittelung über alle Wellenlängen des Spektralbereichs und/oder als ein minimaler Transmissionswert von Licht mit einer innerhalb des Spektralbereichs liegenden Wellenlänge ausgebildet. Zusätzlich kann insbesondere bei einer Berechnung des durchschnittlichen Transmissionswerts ein, insbesondere wellenlängenabhängiger, Gewichtungsfaktor, wie beispielsweise in den Normen ISO 16321-1 und/oder ISO 16321-2 beschrieben, Anwendung finden.

Wenn der optische Blendschutzfilter zumindest eine weitere Flüssigkristallzelle aufweist, welche insbesondere in einer Sichtrichtung durch den optischen Blendschutzfilter hinter und/oder vor einer Flüssigkristallzelle angeordnet ist, kann vorteilhaft eine hohe Flexibilität erreicht werden, insbesondere indem eine große Bandbreite an Schutzstufen abgedeckt werden kann. Zudem kann vorteilhaft eine Sicherheit erhöht werden, beispielsweise durch eine Abfederung einer Fehlfunktion einer Flüssigkristallzelle mit einer entsprechenden Steuerung und/oder Regelung der anderen Flüssigkristallzelle. Die weitere Flüssigkristallzelle ist insbesondre identisch zu der Flüssigkristallzelle ausgebildet. Alternativ kann die weitere Flüssigkristallzelle einen von der Flüssigkristallzelle verschiedenen Flüssigkristallzellentyp ausbilden. Vorzugsweise ist die Steuerung und/oder Regelung der Flüssigkristallzelle und der weiteren Flüssigkristallzelle direkt und/oder indirekt gekoppelt. Insbesondere ist die Flüssigkristallzelle und/oder die weitere Flüssigkristallzelle dazu vorgesehen, zumindest UV-Licht, zumindest sichtbares Licht und/oder zumindest Infrarotlicht zumindest zu einem Teil zu filtern, insbesondere zu absorbieren. Es ist denkbar, dass die Flüssigkristallzelle und die weitere Flüssigkristallzelle zumindest im Wesentlichen identische Filterkurven aufweisen. Alternativ könnten die Flüssigkristallzelle und die weitere Flüssigkristallzelle zumindest im Wesentlichen verschiedene Filterkurven aufweisen. Unter einer "Filterkurve" soll insbesondere eine Wellenlängenabhängigkeit der Transmission durch die Flüssigkristallzelle verstanden werden.

Erfindungsgemäß weist der optische Blendschutzfilter zumindest eine passive Filtereinheit auf. Dadurch kann vorteilhaft eine hohe Sicherheit erreicht werden, insbesondere indem die passive Filtereinheit unabhängig von einer Ansteuerung durch die Steuer- und/oder Regeleinheit zumindest einen Teil des Lichtspektrums, insbesondere einen unsichtbaren und/oder augenschädlichen Teil des Lichtspektrums, wie beispielsweise UV-Strahlung, effektiv absorbiert und/oder reflektiert. Dadurch kann vorteilhaft ein gutes "Fail-Safe"-Verhalten des optischen Blendschutzfilters erreicht werden. Insbesondere genügt die passive Filtereinheit den Anforderungen der Normen ISO 16321-1 und ISO 16321-2. Unter einer "passiven Filtereinheit" soll insbesondere eine Filtereinheit verstanden werden, welche elektromagnetische Strahlung, insbesondere passiv, filtert, absorbiert und/oder reflektiert, wobei Filtereigenschaften, Absorptionseigenschaften und/oder Reflexionseigenschaften nach Montage der passiven Filtereinheit in der Schutzkassette von außen unbeeinflussbar, unsteuerbar und/oder unregelbar sind. Insbesondere absorbiert und/oder reflektiert die passive Filtereinheit zumindest teilweise Licht zumindest eines Teils eines infraroten Spektralbereichs, eines Teils eines sichtbaren Spektralbereichs und/oder eines Teils eines ultravioletten Spektralbereichs elektromagnetischer Strahlung zu zumindest 5 %, vorzugsweise zumindest 10 %, bevorzugt zumindest 15 %, vorteilhaft zumindest 20 %, bevorzugt zumindest 25 % und besonders bevorzugt höchstens 90 %. Vorzugsweise sind die Filtereigenschaften, Absorptionseigenschaften und/oder Reflexionseigenschaften der Bauteile der passiven Filtereinheit, insbesondere abgesehen von Materialkorrosion, zeitlich konstant und/oder unveränderbar. Insbesondere sind die Filtereigenschaften, Absorptionseigenschaften und/oder Reflexionseigenschaften vorwiegend abhängig von, insbesondere konstanten, Materialeigenschaften der jeweiligen Bauteile und/oder von einem Einfallwinkel auftreffender Strahlen. Alternativ oder zusätzlich ist vorstellbar, dass die entsprechend niedrige Schutzstufe in der
Hellstufe, insbesondere unter Einhaltung von Augenschutzbestimmungen wie beispielsweise in zumindest einer der Normen ISO 16321-1, ISO 16321-2, DIN EN 166:2002-04 und/oder DIN EN 169:2003-02 gefordert, mittels einer, insbesondere von den Flüssigkristallzellen verschiedenen und/oder zusätzlich zu den Flüssigkristallzellen vorhandenen, aktiven Filtereinheit erreicht werden kann. Die aktive Filtereinheit kann insbesondere als ein Transparenzelement, insbesondere eine Scheibe und/oder ein electrochromic device (ECD), welche zumindest ein elektrochromes Material umfasst ausgebildet sein, beispielsweise als ein intelligentes Glas, welches auf Spannungen und/oder elektromagnetische Felder reagiert, insbesondere seine Transparenz variiert. Insbesondere könnte ein elektrochromes Material zumindest ein elektrochromes, elektroaktives und/oder elektrochemisches Polymer, wie beispielsweise elektroaktives Polyamid und/oder Heptyl-Viologen, umfassen. Alternativ oder zusätzlich könnte die passive Filtereinheit zumindest eine Absorptionsfolie, eine Metallbeschichtung und/oder eine metallorganische Beschichtung zu einer Beeinflussung der Absorptionseigenschaften und/oder Reflexionseigenschaften der passiven Filtereinheit umfassen.

Erfindungsgemäß umfasst die passive Filtereinheit zumindest ein dichroitisches Filterelement. Dadurch können vorteilhaft verbesserte Eigenschaften hinsichtlich einer Durchlässigkeit, insbesondere ausgewählter Wellenlängenbereiche elektromagnetischer Strahlung, erreicht werden. Insbesondere können vorteilhaft Hellstufen des optischen Blendschutzfilters mit niedrigen Schutzstufen ermöglicht werden. Vorteilhaft kann eine Durchlässigkeit der passiven Filtereinheit auf einen bestimmten Teil des Spektrums eingeschränkt werden, wodurch vorteilhaft eine hohe Sicherheit und/oder ein gutes "Fail-Safe"-Verhalten erreicht werden kann. Das dichroitische Filterelement ist insbesondere als zumindest ein dichroitischer Spiegel und/oder ein Interferenzfilter ausgebildet. Vorzugsweise ist das dichroitische Filterelement als zumindest eine Beschichtung eines, insbesondere zumindest teilweise transparenten, Substrats ausgebildet. Insbesondere umfasst die Beschichtung zumindest eine Mehrzahl an senkrecht zu dem Substrat übereinanderliegenden Teilschichten. Insbesondere könnte die Beschichtung, vorzugsweise zumindest eine, bevorzugt zumindest zwei Teilschichten der Beschichtung, zumindest teilweise aus einem Metall, beispielsweise Silber und/oder Aluminium ausgebildet sein. Insbesondere ist die Beschichtung als zumindest ein Schichtpaket aus zwei dünnen Metallschichten mit einer dazwischenliegenden dielektrischen, transparenten Schicht ausgebildet. Bevorzugt ist die Beschichtung zumindest teilweise als zumindest ein Schichtpaket aus zumindest zwei, vorzugsweise einer Mehrzahl, insbesondere alternierend, senkrecht zu einer Oberfläche des Substrats übereinanderliegenden dielektrischen, nichtmetallischen Schichten mit unterschiedlichen Dicken und unterschiedlichen Brechungsindizes ausgebildet, welches insbesondere zumindest einen Bragg Spiegel ausbildet. Das dichroitische Filterelement ist erfindungsgemäß dazu vorgesehen, zumindest einen Teil der einfallenden elektromagnetischen Strahlung, und zwar den UV-Anteil, insbesondere zumindest zu 95 %, zu reflektieren und/oder zumindest einen, insbesondere weiteren, Teil der einfallenden elektromagnetischen Strahlung, insbesondere den sichtbaren Teil der elektromagnetischen Strahlung, insbesondere zumindest zu 80 %, zu transmittieren. Zudem ist denkbar, dass die passive Filtereinheit zumindest ein weiteres dichroitisches Filterelement umfasst. Dadurch kann vorteilhaft eine wellenlängenabhängige Transmission durch den optischen Blendschutzfilter noch genauer eingestellt werden.

Erfindungsgemäß bildet das dichroitische Filterelement einen Langpassfilter oder einen Bandpassfilter aus. Alternativ ist vorstellbar, dass das dichroitische Filterelement einen Kurzpassfilter ausbildet. Dadurch können vorteilhaft verbesserte Eigenschaften hinsichtlich einer Durchlässigkeit, insbesondere ausgewählter Wellenlängenbereiche elektromagnetischer Strahlung, erreicht werden. Vorteilhaft kann eine Durchlässigkeit der passiven Filtereinheit auf einen bestimmten Teil des Spektrums eingeschränkt werden, wodurch vorteilhaft eine hohe Sicherheit und/oder ein gutes "Fail-Safe"-Verhalten erreicht
werden kann. Unter einem "Langpassfilter" soll insbesondere ein Filter für elektromagnetische Strahlung, welcher elektromagnetische Strahlung mit einer Wellenlänge kleiner als eine Grenzwellenlänge zu einem Großteil reflektiert und elektromagnetische Strahlung mit einer Wellenlänge größer als die Grenzwellenlänge zu einem Großteil transmittiert, verstanden werden. Die Grenzwellenlänge ist insbesondere beliebig wählbar. Vorzugsweise liegt die Grenzwellenlänge am Rand des sichtbaren elektromagnetischen Spektrums, beispielsweise bei 380 nm. Unter einem Großteil soll insbesondere zumindest 80 %, vorzugsweise zumindest 85 %, vorteilhaft zumindest 90 % bevorzugt zumindest 95 % und besonders bevorzugt zumindest 98 % verstanden werden. Unter einem "Bandpassfilter" soll insbesondere ein Filter für elektromagnetische Strahlung, welcher elektromagnetische Strahlung mit einer Wellenlänge kleiner als eine erste Grenzwellenlänge, sowie elektromagnetische Strahlung mit einer Wellenlänge größer als eine von der Grenzwellenlänge verschiedene zweite Grenzwellenlänge zu einem Großteil reflektiert und elektromagnetische Strahlung mit einer Wellenlänge zwischen der ersten Grenzwellenlänge und der zweiten Grenzwellenlänge zu einem Großteil transmittiert, verstanden werden. Die erste Grenzwellenlänge und die zweite Grenzwellenlänge sind insbesondere beliebig wählbar. Vorzugsweise liegen die erste Grenzwellenlänge und die zweite Grenzwellenlänge jeweils an den Rändern des sichtbaren elektromagnetischen Spektrums, beispielsweise bei 380 nm und bei 780 nm.

Erfindungsgemäß umfasst die passive Filtereinheit zumindest ein absorptives und/oder reflektives Infrarotfilterelement, insbesondere zumindest ein Absorptionsglas. Dadurch können vorteilhaft verbesserte Eigenschaften hinsichtlich einer Durchlässigkeit, insbesondere ausgewählter Wellenlängenbereiche elektromagnetischer Strahlung, erreicht werden. Insbesondere können vorteilhaft Hellstufen des optischen Blendschutzfilters mit niedrigen Schutzstufen ermöglicht werden. Vorteilhaft kann eine Durchlässigkeit der passiven Filtereinheit auf einen bestimmten Teil des Spektrums eingeschränkt werden, wodurch vorteilhaft eine hohe Sicherheit und/oder ein gutes "Fail-Safe"-Verhalten erreicht werden kann. Zudem kann dadurch vorteilhaft unsichtbares und/oder augengefährdendes Infrarotlicht zu einem Großteil von einem Benutzerauge ferngehalten werden. Das Infrarotfilterelement ist insbesondere dazu vorgesehen, elektromagnetische Strahlung in einem Wellenlängenbereich ab 780 nm und darüber zu einem Großteil zu absorbieren und/oder zu reflektieren. Ein Absorptionsglas umfasst insbesondere ein, insbesondere eingefärbtes, Glas, welchem insbesondere ein Farbstoff beigemischt ist, welcher insbesondere dazu vorgesehen ist, infrarote Strahlung zu absorbieren. Der Farbstoff umfasst beispielsweise Eisen(II)-, Eisen(III)- und/oder Kupfer(II)-oxid. Insbesondere kann das Infrarotfilterelement einen Kurzpassfilter ausbilden. Insbesondere kann das Infrarotfilterelement, insbesondere das Absorptionsglas, nur zu einem Teil aus dem eingefärbtem Glas ausgebildet sein. Beispielsweise könnte das Infrarotfilterelement, insbesondere das Absorptionsglas, eine Schichtstruktur aufweisen, wobei insbesondere eine Schicht eine hohe Absorption infraroter Strahlung verursacht. Einzelne Schichten der Schichtstruktur können insbesondere stoffschlüssig verbunden sein. Unter "stoffschlüssige verbunden" soll insbesondere verstanden werden, dass die Masseteile durch atomare oder molekulare Kräfte zusammengehalten werden, wie beispielsweise beim Kleben, Laminieren und/oder Vulkanisieren. Alternativ oder zusätzlich kann das Infrarotfilterelement, insbesondere das Absorptionsglas, eine aufgedampfte, insbesondere gesputterte, und/oder durch ein weiteres Beschichtungsverfahren aufgebrachte Beschichtung aufweisen, welche dazu vorgesehen ist, infrarote Strahlung zu absorbieren. Zudem ist vorstellbar, dass einzelne Schichten der Schichtstruktur des Infrarotfilterelements, insbesondere des Absorptionsglases, und/oder eine Verbindungsschicht des Infrarotfilterelements, insbesondere des Absorptionsglases, zu einer Verbindung des Infrarotfilterelements, insbesondere des Absorptionsglases, mit zumindest einem an das Infrarotfilterelement, insbesondere das Absorptionsglas, angrenzenden Element, beispielsweise dem dichroitischen Filterelement, mittels einem Kleber verbunden sind, welcher insbesondere eine Schicht ausbildet, die infrarote Strahlung effizient absorbiert und somit vorzugsweise das Infrarotfilterelement, insbesondere das Absorptionsglas, zumindest teilweise ausbildet. Insbesondere kann das Infrarotfilterelement in Kombination mit einem, einen Langpassfilter ausbildenden dichroitischen Spiegel einen Bandpassfilter ausbilden.

Ferner wird vorgeschlagen, dass die passive Filtereinheit in zumindest einem, insbesondere sichtbaren, vorzugsweise eine begrenzte Bandbreite ausbildenden, Spektralbereich eine Transmissivität von zumindest 80 %, vorzugsweise zumindest 85 %, bevorzugt zumindest 90 % und besonders bevorzugt höchstens 95 % aufweist. Dadurch können vorteilhaft verbesserte Eigenschaften hinsichtlich einer Durchlässigkeit, insbesondere ausgewählter Wellenlängenbereiche elektromagnetischer Strahlung, erreicht werden. Außerdem kann vorteilhaft ein hoher Benutzerkomfort erreicht werden, insbesondere indem einem Benutzer in der Hellstufe eine für eine Orientierung im Raum ausreichende Sicht durch den optischen Blendschutzfilter bereitgestellt werden kann. Vorteilhaft kann dadurch zudem eine Sicherheit für den Benutzer erhöht werden. Insbesondere umfasst die "begrenzte Bandbreite" einen Wellenlängenbereich, welcher oberhalb 380 nm, vorzugsweise oberhalb 420 nm und bevorzugt oberhalb 470 nm, sowie unterhalb 550 nm, bevorzugt unterhalb 590 nm und besonders bevorzugt unterhalb 780 nm liegt.

Weiterhin wird vorgeschlagen, dass die passive Filtereinheit in zumindest einem, insbesondere begrenzten, vorzugsweise ultravioletten, weiteren Spektralbereich eine Transmissivität von höchstens 5 %, vorzugsweise höchstens 2 %, bevorzugt höchstens 1 % und besonders bevorzugt 0 % aufweist. Dadurch kann vorteilhaft eine hohe Sicherheit erreicht werden, insbesondere indem augengefährdende und/oder unsichtbare UV-Strahlung von einem Benutzerauge ferngehalten werden kann. Insbesondere umfasst der weitere Spektralbereich Licht mit Wellenlängen zumindest kleiner als 470 nm, vorzugsweise zumindest kleiner als 420 nm, bevorzugt zumindest kleiner als 380 nm und besonders bevorzugt zumindest kleiner als 350 nm.

Erfindungsgemäß weist der optische Blendschutzfilter zumindest eine Antireflexionseinheit auf. Dadurch können vorteilhaft verbesserte Eigenschaften hinsichtlich einer Durchlässigkeit erreicht werden. Erfindungsgemäß ist die Antireflexionseinheit auf einer im getragenen Zustand einem Benutzerauge zugewandten Seite des optischen Blendschutzfilters angeordnet. Alternativ oder zusätzlich kann die Antireflexionseinheit auf einer im getragenen Zustand dem Benutzerauge abgewandten Seite des optischen Blendschutzfilters angeordnet sein. Die Antireflexionseinheit ist dazu vorgesehen, in zumindest einem Spektralbereich, vorzugsweise in einem sichtbares Licht umfassenden Spektralbereich, welcher insbesondere durch Wellenlängen oberhalb 380 nm, vorzugsweise oberhalb 420 nm und bevorzugt oberhalb 470 nm und/oder unterhalb 550 nm, bevorzugt unterhalb 590 nm und besonders bevorzugt unterhalb 780 nm begrenzt ist, eine Reduzierung einer Reflexion zu bewirken, welche insbesondere in dem Spektralbereich eine Steigerung einer Transmissivität des optischen Blendschutzfilters um zumindest 2 %, vorzugsweise um zumindest 3 % und bevorzugt um zumindest 4 % bewirkt. Die Antireflexionseinheit umfasst insbesondere zumindest einen Interferenzfilter, insbesondere mit zumindest einer Vergütungsschicht und/oder mit zumindest einer Mehrzahl an Vergütungsschichten. Alternativ oder zusätzlich kann die Antireflexionseinheit zumindest eine unter Ausnutzung des "Mottenaugen-Effekts" nanostrukturierte Oberfläche, zumindest eine brechungsindexreduzierende Oberflächenstruktur und/oder zumindest einen Zirkularpolarisator umfassen.

Ferner wird eine Blendschutzvorrichtung mit einer Schutzkassette vorgeschlagen. Dadurch kann vorteilhaft ein hoher Benutzerkomfort erreicht werden, insbesondere indem einem Benutzer in der Hellstufe eine für eine Orientierung im Raum ausreichende Sicht durch den optischen Blendschutzfilter bereitgestellt werden kann. Vorteilhaft kann eine Sicherheit erhöht werden, insbesondere indem eine Notwendigkeit eines temporären Entfernens des optischen Blendschutzfilters aus einem Sichtfeld eines Benutzers zu einer Orientierung vermieden werden kann und/oder indem eine gefahrlose Orientierung im Raum mit dem optischen Blendschutzfilter in dem Sichtfeld des Benutzers ermöglicht werden kann. Zudem kann, beispielsweise bei einem Einsatz des optischen Blendschutzfilters zu Schweißarbeiten, vorteilhaft eine Arbeitserleichterung für einen Benutzer erreicht werden, insbesondere indem ein Ergebnis eines, insbesondere Licht emittierenden, Arbeitsschrittes direkt im Anschluss an die Lichtemission, insbesondere ohne ein Entfernen des optischen Blendschutzfilters aus dem Blickfeld des Benutzers, kontrolliert werden kann. Dadurch kann vorteilhaft eine hohe Arbeitsgeschwindigkeit erreicht werden.

Außerdem wird ein Verfahren mit einer Schutzkassette vorgeschlagen. Dadurch können vorteilhaft verbesserte Eigenschaften hinsichtlich einer Durchlässigkeit von Licht erreicht werden.

Zudem wird ein Verfahren zur Herstellung einer Schutzkassette vorgeschlagen, wobei in zumindest einem Verfahrensschritt ein dichroitisches Filterelement, insbesondere mittels chemischer Gasphasenabscheidung, auf ein, insbesondere zumindest teilweise als ein Absorptionsglas ausgebildetes, Substrat aufgedampft wird. Dadurch können vorteilhaft verbesserte Eigenschaften hinsichtlich einer Durchlässigkeit erreicht werden. Alternativ oder zusätzlich kann das dichroitische Filterelement mittels physikalischer Gasphasenabscheidung und/oder Kathodenzerstäubung auf das Substrat, insbesondere das Absorptionsglas, aufgebracht werden.

Die erfindungsgemäße Schutzkassette und/oder das erfindungsgemäße Verfahren soll/sollen hierbei nicht auf die oben beschriebene Anwendung und Ausführungsform beschränkt sein. Insbesondere kann die erfindungsgemäße Schutzkassette und/oder das erfindungsgemäße Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine perspektivische schematische Ansicht einer Blendschutzvorrichtung mit einer Schutzkassette,
- Fig. 2: eine schematische Schnittansicht der Blendschutzvorrichtung mit der Schutzkassette im getragenen Zustand,
- Fig. 3: eine schematische Seitenansicht in einer Explosionsdarstellung eines optischen Blendschutzfilters der Schutzkassette,
- Fig. 4: ein Transmissivitäts-Wellenlängen-Diagramm eines Teils des optischen Blendschutzfilters und
- Fig. 5: ein Ablaufdiagramm eines Verfahrens mit der Schutzkassette und eines Verfahrens zur Herstellung der Schutzkassette.

### Beschreibung des Ausführungsbeispiels

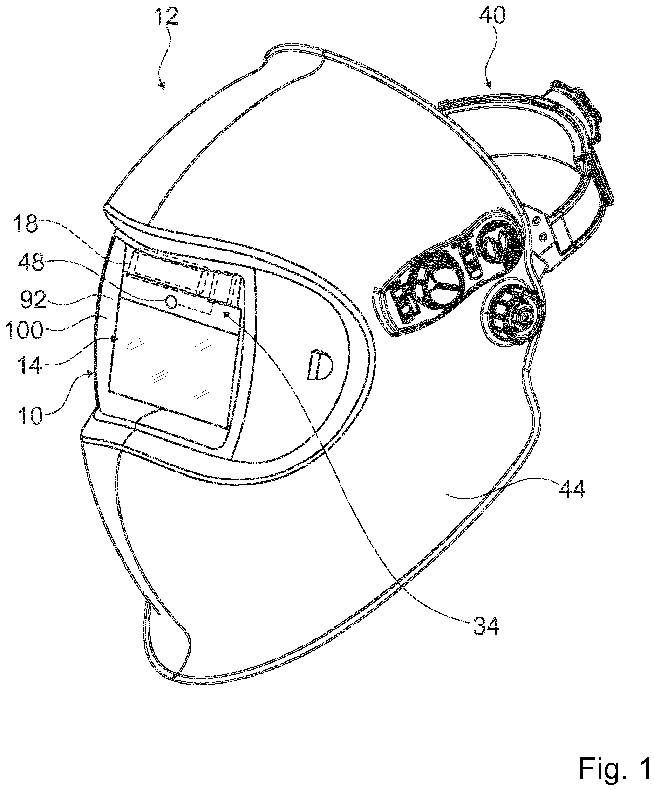

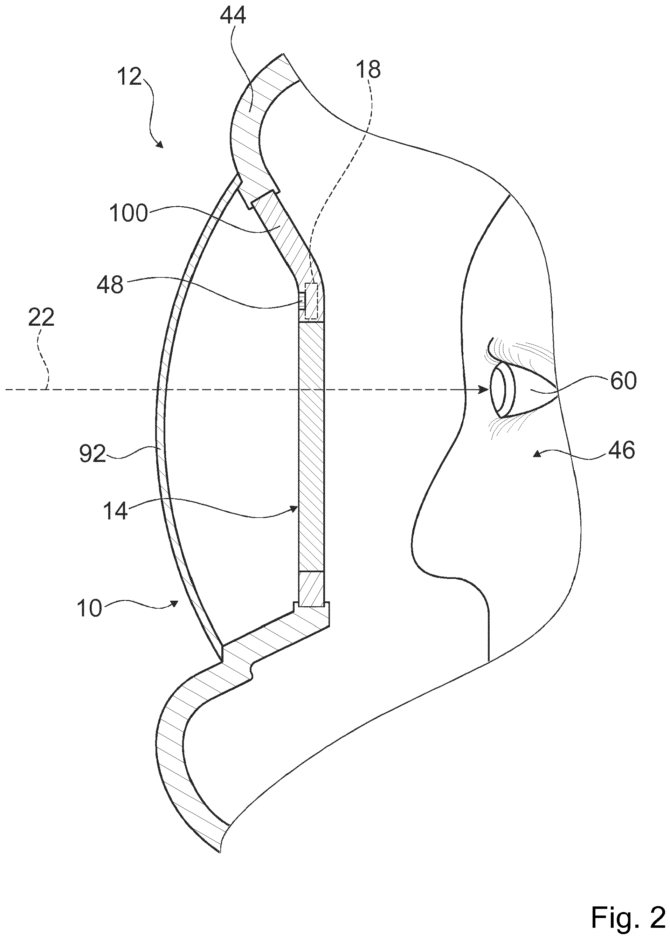

Fig. 1 zeigt eine Blendschutzvorrichtung 12. Die Blendschutzvorrichtung 12 ist als ein Schweißhelm ausgebildet. Grundsätzlich wäre jedoch auch eine andere, einem Fachmann als sinnvoll erscheinende Ausbildung der Blendschutzvorrichtung 12 denkbar. Die Blendschutzvorrichtung 12 ist dazu vorgesehen, während eines Betriebs von einem Bediener auf dem Kopf getragen zu werden (vgl. auch Fig. 2). Der Bediener ist hierbei von einem Träger gebildet. Die Blendschutzvorrichtung 12 weist einen Grundkörper 44 auf. Der Grundkörper 44 umschließt ein Gesicht 46 eines Benutzers. Der Grundkörper 44 bildet ein lichtundurchlässiges, das Gesicht 46 des Benutzers abschirmendes Schild aus. Der Grundkörper 44 ist aus einem hitzebeständigen, schwer entflammbaren Material, beispielsweise Polyamid, ausgebildet. Die Blendschutzvorrichtung 12 weist eine Kopfbefestigungseinheit 40 auf. Die Kopfbefestigungseinheit 40 ist zu einer Befestigung an dem Kopf des Bedieners vorgesehen. Die Kopfbefestigungseinheit 40 ist von einem Kopfband gebildet. Die Kopfbefestigungseinheit 40 ist nicht weiter sichtbar mit dem Grundkörper 44 verbunden.

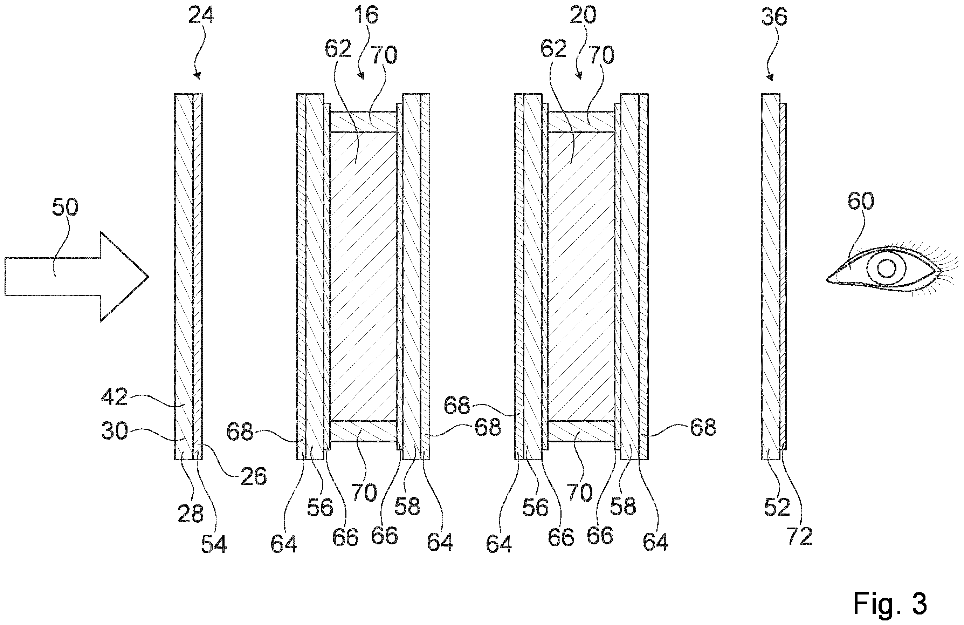

Die Blendschutzvorrichtung 12 weist eine Schutzkassette 10 auf. Die Schutzkassette 10 weist einen optischen Blendschutzfilter 14 auf. Die Schutzkassette 10 weist eine Vorsatzscheibe 92 auf. Die Vorsatzscheibe 92 ist zu einem Schutz des optischen Blendschutzfilters 14 vorgesehen. Es ist denkbar, dass die Vorsatzscheibe 92 antireflexionsbeschichtet ausgebildet ist. Der optische Blendschutzfilter 14 weist eine Flüssigkristallzelle 16 auf. Der optische Blendschutzfilter 14 weist eine weitere Flüssigkristallzelle 20 auf (vgl. Fig. 2 und 3). Die Flüssigkristallzelle 16 und die weitere Flüssigkristallzelle 20 weisen unterschiedliche Außenmaße auf. Alternativ könnte die Flüssigkristallzelle 16 und die weitere Flüssigkristallzelle 20 zumindest im Wesentlichen identische Außenmaße aufweisen. Die Flüssigkristallzelle 16 und die weitere Flüssigkristallzelle 20 sind in einer Sichtrichtung 22 durch den optischen Blendschutzfilter 14 hintereinander angeordnet. Die Sichtrichtung 22 ist als eine die Schutzkassette 10 und ein Auge 60 eines Benutzers verbindende, senkrecht auf einer Oberfläche der Schutzkassette 10 stehende Verbindungslinie ausgebildet. Die Flüssigkristallzelle 16 und die weitere Flüssigkristallzelle 20 liegen berührend aneinander an. Die Flüssigkristallzelle 16 und die weitere Flüssigkristallzelle 20 sind miteinander verklebt. Die Flüssigkristallzelle 16 und die weitere Flüssigkristallzelle 20 sind als "normally white" TN-Flüssigkristallzellen ausgebildet. Grundsätzlich wäre jedoch auch eine andere, einem Fachmann als sinnvoll erscheinende Ausbildung der Flüssigkristallzellen 16, 20 denkbar.

Ferner weist die Schutzkassette 10 eine Steuer- und Regeleinheit 18 auf. Die Steuer- und Regeleinheit 18 ist dazu vorgesehen, abhängig von einem erfassten Arbeitszustand und einer Lichteinstrahlung eine Durchlässigkeit des optischen Blendschutzfilters 14 zu steuern. Die Steuer- und Regeleinheit 18 ist dazu vorgesehen, abhängig von einem erfassten Arbeitszustand, einem elektronischen Signal einer externen Signalquelle oder einer Lichteinstrahlung eine Durchlässigkeit des optischen Blendschutzfilters 14 zu regeln. Die Schutzkassette 10 weist eine Sensoreinheit 34 auf. Die Steuer- und Regeleinheit 18 ist mit der Sensoreinheit 34 verbunden. Die Sensoreinheit 34 ist zu einer Erfassung eines Arbeitszustands und/oder einer Lichteinstrahlung vorgesehen. Die Sensoreinheit 34 weist zumindest einen Sensor 48 auf. Der Sensor 48 ist dazu vorgesehen, einen Schweißvorgang oder das Auftreten eines hellen Lichts, welches die Augen 60 eines Benutzers schädigen oder auf andere Weise beeinflussen könnte, zu detektieren. Der Sensor 48 der Sensoreinheit 34 ist von einer Fotodiode gebildet. Grundsätzlich wäre jedoch auch eine andere, einem Fachmann als sinnvoll erscheinende Ausbildung des Sensors 48 der Sensoreinheit 34 denkbar.

Die Schutzkassette 10 weist ein Gehäuse 100 auf. Der optische Blendschutzfilter 14 ist in dem Gehäuse 100 teilweise aufgenommen. Die Steuer- und Regeleinheit 18 ist in dem Gehäuse 100 aufgenommen. Die Sensoreinheit 34 ist in dem Gehäuse 100 aufgenommen. Der Sensor 48 der Sensoreinheit 34 ist, während eines Betriebs der Blendschutzvorrichtung 12, teilweise auf einer einem Gesicht 46 des Benutzers abgewandten Außenseite der Schutzkassette 10 angeordnet. Das Gehäuse 100 der Schutzkassette 10 ist in die Blendschutzvorrichtung 12 einsetzbar und/oder aus der Blendschutzvorrichtung 12 entnehmbar. Dadurch kann vorteilhaft ein einfacher Austausch zu einer Reparatur, einer Wartung und/oder einem Wechsel der Schutzkassette 10 ermöglicht werden.

Die Steuer- und Regeleinheit 18 ist dazu vorgesehen, Daten der Sensoreinheit 34 zu verarbeiten und abhängig davon den optischen Blendschutzfilter 14 und/oder die Flüssigkristallzelle 16 und die weitere Flüssigkristallzelle 20 anzusteuern. Der optische Blendschutzfilter 14 weist eine Durchlässigkeit für elektromagnetische Strahlung auf. Die Durchlässigkeit des optischen Blendschutzfilters 14 für elektromagnetische Strahlung ist regelbar und/oder steuerbar. Die Steuer- und Regeleinheit 18 ist dazu vorgesehen, den optischen Blendschutzfilter 14 zwischen einer Hellstufe und Dunkelstufen zu steuern. Die Steuer- und Regeleinheit 18 ist dazu vorgesehen, den optischen Blendschutzfilter 14 zwischen einer Hellstufe und verschiedenen Dunkelstufen zu regeln.

Die Durchlässigkeit des optischen Blendschutzfilters 14 in der Hellstufe entspricht einer Schutzstufe kleiner als 2,5. Die Durchlässigkeit des optischen Blendschutzfilters 14 in der Hellstufe entspricht einer Schutzstufe größer als 1,7. Die Durchlässigkeit des optischen Blendschutzfilters 14 in einer der Dunkelstufen entspricht einer Schutzstufe von 3. Eine minimale Durchlässigkeit des optischen Blendschutzfilters 14 in einer der Dunkelstufen entspricht einer Schutzstufe von 16. Der optische Blendschutzfilter 14 weist in der Hellstufe in einem Spektralbereich sichtbaren Lichts eine Transmissivität 32 von zumindest 29 % auf.

Fig. 3 zeigt eine schematische Seitenansicht des optischen Blendschutzfilters 14. Eine typische Einfallrichtung 50 für abzuschirmende elektromagnetische Strahlung ist in Pfeilrichtung angezeigt. Eine Position eines Benutzers ist auf einer der Einfallrichtung 50 gegenüberliegenden Seite des optischen Blendschutzfilters 14 durch ein Auge 60 angedeutet. Der optische Blendschutzfilter 14 weist eine Abdeckscheibe 30 auf. Der optische Blendschutzfilter 14 weist eine weitere Abdeckscheibe 52 auf. Die Flüssigkristallzelle 16 und die weitere Flüssigkristallzelle 20 sind in Sichtrichtung 22 zwischen der Abdeckscheibe 30 und der weiteren Abdeckscheibe 52 angeordnet. Die Abdeckscheibe 30 ist auf einer dem Benutzer im getragenen Zustand abgewandten Seite des optischen Blendschutzfilters 14 angeordnet. Die weitere Abdeckscheibe 52 ist auf einer dem Benutzer im getragenen Zustand zugewandten Seite des optischen Blendschutzfilters 14 angeordnet. Die Abdeckscheibe 30 und die weitere Abdeckscheibe 52 sind zu einem Schutz der Flüssigkristallzelle 16 und der weiteren Flüssigkristallzelle 20 und deren Bestandteile vorgesehen.

Der optische Blendschutzfilter 14 weist ein Transparenzelement 56 auf. Der optische Blendschutzfilter 14 weist ein weiteres Transparenzelement 58 auf. Die Transparenzelemente 56, 58 sind als Glasscheiben ausgebildet. Die Flüssigkristallzellen 16, 20 sind in Sichtrichtung 22 jeweils beidseitig von Transparenzelementen 56, 58 begrenzt. Die Transparenzelemente 56, 58 sind beschichtet. Die Transparenzelemente 56, 58 weisen jeweils auf einer Oberfläche eine Beschichtung 66 auf. Die Beschichtung 66 der Transparenzelemente 56, 58 ist als eine Indium-Zinn-Oxidschicht (ITO) ausgebildet. Alternativ oder zusätzlich kann die Beschichtung 66 zumindest teilweise als eine AZO-Beschichtung und/oder eine Si-Nanowire-Beschichtung ausgebildet sein und/oder die Transparenzelemente 56, 58 können zumindest eine weitere Beschichtung aus einem der vorgenannten Materialien umfassen. Grundsätzlich sind jedoch auch noch weitere einem Fachmann als sinnvoll erscheinende Ausbildungen und/oder Ausgestaltungen der Beschichtung 66 denkbar. Die Beschichtung 66 der Transparenzelemente 56, 58 ist dazu vorgesehen eine transparente Elektrodenschicht auszubilden. Die Beschichtung 66 der Transparenzelemente 56, 58 ist auf den einem Inneren der jeweiligen Flüssigkristallzelle 16, 20 zugewandten Oberflächen der Transparenzelemente 56, 58 angeordnet.

Die Transparenzelemente 56, 58 weisen jeweils auf einer weiteren Oberfläche eine weitere Beschichtung 64 auf. Die Beschichtung 66 der Transparenzelemente 56, 58 und die weitere Beschichtung 64 der Transparenzelemente 56, 58 sind auf gegenüberliegenden Oberflächen der Transparenzelemente 56, 58 angeordnet. Die Flüssigkristallzellen 16, 20 weisen Polarisationselemente 68 auf. Die Polarisationselemente 68 sind einstückig mit der weiteren Beschichtung 64 der Transparenzelemente 56, 58 ausgebildet. Die weitere Beschichtung 64 bildet ein Polarisationselement 68 aus. Die Polarisationselemente 68 sind jeweils auf der dem Inneren der jeweiligen Flüssigkristallzelle 16, 20 abgewandten Oberfläche der Transparenzelemente 56, 58 angeordnet. Die Polarisationselemente 68 sind als Polarisationsfilter ausgebildet. Die Polarisationselemente 68 weisen eine Polarisationsvorzugsrichtung auf. Die Polarisationselemente 68 sind dazu vorgesehen, Licht einer Polarisationsvorzugsrichtung aus dem einfallenden Licht herauszufiltern, insbesondere zu transmittieren. Die Polarisationsvorzugsrichtungen der Polarisationselemente 68 der Flüssigkristallzellen 16, 20 sind gekreuzt, insbesondere senkrecht, zueinander ausgerichtet. Alternativ ist denkbar, dass zumindest eine oder mehrere Polarisationselemente 68 zumindest teilweise verschiedene, beispielsweise senkrecht zueinander ausgerichtete, Polarisationsvorzugsrichtungen aufweisen.

Die Flüssigkristallzellen 16, 20 weisen jeweils eine aktive Schicht 62 auf. Die aktive Schicht 62 ist dazu vorgesehen, eine Polarisationsrichtung einfallenden Lichts mittels einer Ansteuerung durch die Steuer und/oder Regeleinheit 18 zu manipulieren. Die aktive Schicht 62 ist dazu vorgesehen, abhängig von einer anliegenden Spannung die Polarisationsrichtung von Lichtstrahlen zu drehen. Die aktive Schicht 62 ist zumindest teilweise flüssig. Die aktive Schicht 62 ist als eine Flüssigkristallschicht ausgebildet. Die aktive Schicht 62 ist in Sichtrichtung 22 durch die Beschichtungen 66 der Transparenzelemente 56, 58 begrenzt. Die Flüssigkristallzellen 16, 20 weisen jeweils Randabdichtungen 70 auf. Die Randabdichtungen 70 sind dazu vorgesehen, die aktive Schicht 62 in Richtungen senkrecht zu den Oberflächen der Transparenzelemente 56, 58 zu begrenzen. Die Randabdichtungen 70 sind dazu vorgesehen, die aktive Schicht 62 in dem Inneren der Flüssigkristallzellen 16, 20 zu halten.

Der optische Blendschutzfilter 14 weist eine passive Filtereinheit 24 auf. Die passive Filtereinheit 24 weist ein absorptives Infrarotfilterelement 28 auf. Das Infrarotfilterelement 28 ist als ein Absorptionsglas ausgebildet. Das Infrarotfilterelement 28 ist einstückig mit der Abdeckscheibe 30 ausgebildet. Die passive Filtereinheit 24 umfasst ein dichroitisches Filterelement 26. Das dichroitische Filterelement 26 bildet einen Langpassfilter aus. Alternativ kann das dichroitische Filterelement 26 einen Bandpassfilter ausbilden. In Kombination bilden das dichroitische Filterelement 26 und das Infrarotfilterelement 28 einen Bandpassfilter aus. Das dichroitische Filterelement 26 ist als eine Beschichtung 54 ausgebildet. Die Beschichtung 54 ist als ein Schichtpaket dielektrischer Schichten ausgebildet. Das dichroitische Filterelement 26 ist stoffschlüssig, insbesondere haftend mit der Abdeckscheibe 30 verbunden. Das dichroitische Filterelement 26 ist auf einer dem Benutzer im getragenen Zustand zugewandten Seite der Abdeckscheibe 30 angeordnet. Das dichroitische Filterelement 26 ist auf einer der Flüssigkristallzelle 16 zugewandten Seite der Abdeckscheibe 30 angeordnet. Alternativ oder zusätzlich kann das dichroitische Filterelement 26 auch auf einer dem Benutzer im getragenen Zustand abgewandten Seite der Abdeckscheibe und/oder auf beiden Seiten der Abdeckscheibe angeordnet sein.

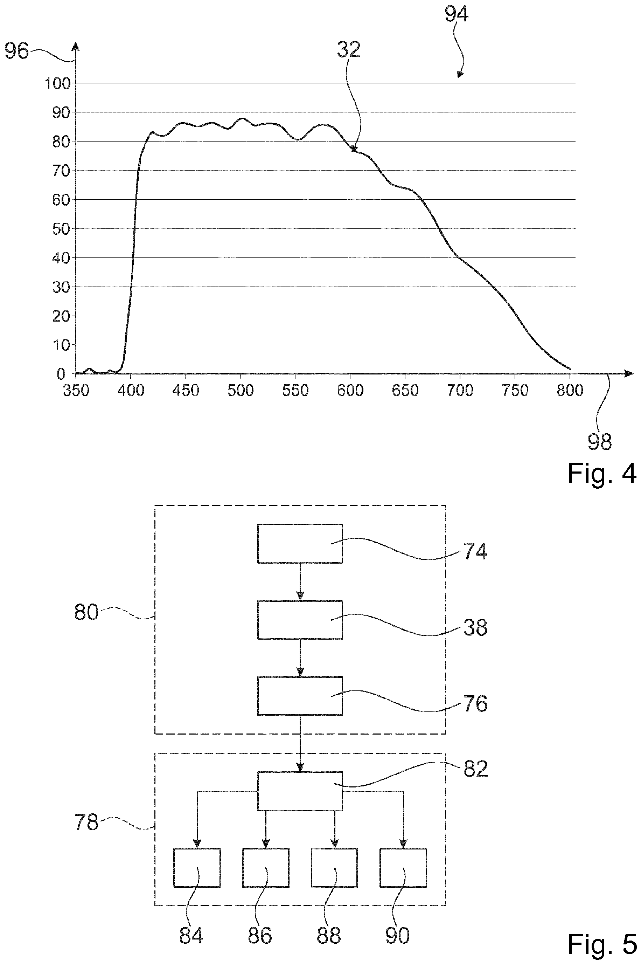

Das dichroitische Filterelement 26 weist in einem sichtbaren Spektralbereich eine Transmissivität 32 von mehr als 80 % auf. Das Infrarotfilterelement 28 weist in einem sichtbaren Spektralbereich eine Transmissivität 32 von mehr als 80 % auf. Die passive Filtereinheit 24 weist in einem sichtbaren Spektralbereich mit Wellenlängen zwischen 410 nm und 590 nm eine Transmissivität 32 von mehr als 80 % auf (vgl. Fig. 4). Das Infrarotfilterelement 28 weist in einem infraroten Spektralbereich eine Transmissivität 32 von weniger als 2 % auf. Als "infraroter Spektralbereich" soll insbesondere ein Wellenlängenbereich elektromagnetischer Strahlung verstanden werden, welcher sich zwischen den Wellenlängen 780 nm und 3000 nm erstreckt. Die passive Filtereinheit 24 weist in einem infraroten Spektralbereich mit Wellenlängen oberhalb von 780 nm eine Transmissivität 32 von weniger als 2 % auf (vgl. Fig. 4). Ein Mittelwert einer Transmissivität des gesamten optischen Blendschutzfilters 14, insbesondere der passiven Filtereinheit 24, bei einer Mittelung der Transmissivitäten aller Wellenlängen des infraroten Spektralbereichs beträgt weniger als 1 %. Das dichroitische Filterelement 26 weist in einem ultravioletten Spektralbereich eine Transmissivität 32 von weniger als 2 % auf. Die passive Filtereinheit 24 weist in einem ultravioletten Spektralbereich mit Wellenlängen unterhalb von 390 nm eine Transmissivität 32 von weniger als 2 % auf (vgl. Fig. 4).

Der optische Blendschutzfilter 14 weist eine Antireflexionseinheit 36 auf. Die Antireflexionseinheit 36 weist eine Antireflexbeschichtung 72 auf. Die Antireflexbeschichtung 72 ist auf einer Oberfläche der weiteren Abdeckscheibe 52 angeordnet. Die Antireflexbeschichtung 72 ist auf der im getragenen Zustand einem Benutzer zugewandten Oberfläche der Abdeckscheibe 52 angeordnet. Die Antireflexbeschichtung 72 ist als ein Interferenzfilter ausgebildet. Grundsätzlich wären jedoch auch andere, einem Fachmann als sinnvoll erscheinende Ausbildungen der Antireflexbeschichtung 72 denkbar. Die Antireflexionseinheit 36 ist dazu vorgesehen, eine Transmission durch den optischen Blendschutzfilter 14 zu erhöhen. Die Antireflexionseinheit 36 ist dazu vorgesehen, eine Reflexion an der Oberfläche der weiteren Abdeckscheibe 52 mittels Beeinflussung einer Brechungsindexdifferenz zwischen Luft und Abdeckscheibenmaterial zu reduzieren.

Fig. 4 zeigt ein Transmissivitäts-Wellenlängen-Diagramm 94 der Abdeckscheibe 30 mit dem Infrarotfilterelement 28 und des dichroitischen Filterelements 26. Auf einer Abszisse 98 des Transmissivitäts-Wellenlängen-Diagramms 94 ist eine Wellenlänge elektromagnetischer Strahlung in Nanometer aufgetragen. Auf einer Ordinate 96 des Transmissivitäts-Wellenlängen-Diagramms 94 ist eine Transmissivität 32 durch die Abdeckscheibe 30 mit dem Infrarotfilterelement 28 und durch das dichroitische Filterelement 26 in Prozent aufgetragen. Die Transmissivität 32 gibt einen wellenlängenabhängigen Anteil eines gesamten auf einer Außenseite der Abdeckscheibe 30 einfallenden Lichts an, welches die Abdeckscheibe 30 mit dem Infrarotfilterelement 28 und das dichroitische Filterelement 26 durchdringt und somit insbesondere auf einer der Abdeckscheibe 30 abgewandten Außenseite des dichroitischen Filterelements 26 wieder austritt. Alternativ oder zusätzlich ist die Transmissivität 32 als ein wellenlängenabhängiger Anteil eines gesamten auf der, der Abdeckscheibe 30 abgewandten Außenseite des dichroitischen Filterelements 26 einfallenden Lichts, welcher auf der Außenseite der Abdeckscheibe 30 wieder austritt, zu verstehen. Die Transmissivität 32, bzw. der Wert der Transmissivität 32 ist insbesondere in beide vorgenannte Richtungen gleich groß. Die Transmissivität 32 ist in einem Bereich von Wellenlängen zwischen 410 nm und 590 nm größer als 80 %. Die Transmissivität 32 ist in einem Bereich von Wellenlängen zwischen 400 nm und 680 nm größer als 50 %. Die Transmissivität 32 ist in einem Bereich von Wellenlängen kleiner als 390 nm kleiner als 2 %. Die Transmissivität 32 ist in einem Bereich von Wellenlängen größer als 800 nm kleiner als 2 %.

Fig. 5 zeigt ein Ablaufdiagramm eines Verfahrens 78 mit einer Schutzkassette 10 und eines Verfahrens 80 zur Herstellung einer Schutzkassette 10. Grundsätzlich ist denkbar, dass das Verfahren 78 und/oder das Verfahren 80 weitere in Fig. 5 nicht gezeigte Verfahrensschritte, Verfahrensteilschritte und/oder Verfahrenszwischenschritte aufweist. In zumindest einem Verfahrensschritt 74 wird ein Substrat 42 bereitgestellt. Das Substrat 42 ist als die Abdeckscheibe 30 ausgebildet. In zumindest einem weiteren Verfahrensschritt 38 wird das dichroitische Filterelement 26 auf das Substrat 42 aufgedampft. Das Aufdampfen geschieht mittels chemischer Gasphasenabscheidung. Grundsätzlich wäre jedoch auch eine andere, einem Fachmann als sinnvoll erscheinende Beschichtungsmethode denkbar. In zumindest einem weiteren Verfahrensschritt 76 wird das Substrat 42 mit dem aufgedampften dichroitischen Filterelement 26 in eine Schutzkassette 10 für eine Blendschutzvorrichtung 12 eingebaut. In zumindest einem weiteren Verfahrensschritt 82 wird die Blendschutzvorrichtung 12 mit der Schutzkassette 10 in Betrieb genommen. In zumindest einem weiteren Verfahrensschritt 84 wird die Blendschutzvorrichtung 12 mit einer Hellstufe in der die Durchlässigkeit des optischen Blendschutzfilters 14 einer Schutzstufe kleiner als 2,5 entspricht, betrieben. In zumindest einem weiteren Verfahrensschritt 86 wird die Blendschutzvorrichtung 12 mit einer Hellstufe in der die Durchlässigkeit des optischen Blendschutzfilters 14 einer Schutzstufe kleiner als 2,25 entspricht, betrieben. In zumindest einem weiteren Verfahrensschritt 88 wird die Blendschutzvorrichtung 12 mit einer Hellstufe in der die Durchlässigkeit des optischen Blendschutzfilters 14 einer Schutzstufe kleiner als 2 entspricht, betrieben. In zumindest einem weiteren Verfahrensschritt 90 wird die Blendschutzvorrichtung 12 mit einer Dunkelstufe in der die Durchlässigkeit des optischen Blendschutzfilters 14 einer Schutzstufe von 8 oder größer entspricht, betrieben.

### Bezugszeichen

- 10: Schutzkassette
- 12: Blendschutzvorrichtung
- 14: Optischer Blendschutzfilter
- 16: Flüssigkristallzelle
- 18: Steuer- und/oder Regeleinheit
- 20: Weitere Flüssigkristallzelle
- 22: Sichtrichtung
- 24: Passive Filtereinheit
- 26: Dichroitisches Filterelement
- 28: Infrarotfilterelement
- 30: Abdeckscheibe
- 32: Transmissivität
- 34: Sensoreinheit
- 36: Antireflexionseinheit
- 38: Verfahrensschritt
- 40: Kopfbefestigungseinheit
- 42: Substrat
- 44: Grundkörper
- 46: Gesicht
- 48: Sensor
- 50: Einfallrichtung
- 52: Weitere Abdeckscheibe
- 54: Beschichtung
- 56: Transparenzelement
- 58: Weiteres Transparenzelement
- 60: Auge
- 62: Aktive Schicht
- 64: Weitere Beschichtung
- 66: Beschichtung
- 68: Polarisationselement
- 70: Randabdichtung
- 72: Antireflexbeschichtung
- 74: Verfahrensschritt
- 76: Verfahrensschritt
- 78: Verfahren
- 80: Verfahren
- 82: Verfahrensschritt
- 84: Verfahrensschritt
- 86: Verfahrensschritt
- 88: Verfahrensschritt
- 90: Verfahrensschritt
- 92: Vorsatzscheibe
- 94: Transmissivitäts-Wellenlängen-Diagramm
- 96: Ordinate
- 98: Abszisse
- 100: Gehäuse

## Patentansprüche

1. Schutzkassette für eine Blendschutzvorrichtung (12), mit einem optischen Blendschutzfilter (14), welcher zumindest eine Flüssigkristallzelle (16) aufweist, mit zumindest einer Steuer- und/oder Regeleinheit (18), welche dazu vorgesehen ist, abhängig von einem erfassten Arbeitszustand, einem elektronischen Signal einer externen Signalquelle und/oder einer Lichteinstrahlung eine Durchlässigkeit des optischen Blendschutzfilters (14) zumindest zwischen zumindest einer Hellstufe und zumindest einer Dunkelstufe zu steuern und/oder zu regeln, wobei die Blendschutzvorrichtung (12) zumindest eine Informationsschnittstelle zu einem Empfang zumindest einer Information über zumindest einen Arbeitszustand und/oder zumindest einen Sensor zu einer Detektion zumindest eines Arbeitszustands aufweist, wobei die Durchlässigkeit des optischen Blendschutzfilters (14) in zumindest einer Hellstufe einer Schutzstufe kleiner als 2,5 entspricht, wobei der optische Blendschutzfilter (14) zumindest eine passive Filtereinheit (24) aufweist, wobei die passive Filtereinheit (24) zumindest ein absorptives und/oder reflektives Infrarotfilterelement (28), umfasst, **dadurch gekennzeichnet, dass** die passive Filtereinheit (24) zumindest ein dichroitisches Filterelement (26) umfasst, wobei das dichroitische Filterelement (26) einen Langpassfilter ausbildet, wobei das Infrarotfilterelement (28) einstückig mit einer Abdeckscheibe (30) ausgebildet ist, welche auf einer dem Benutzer im getragenen Zustand abgewandten Seite des optischen Blendschutzfilters (14) angeordnet ist, wobei das dichroitische Filterelement (26) dazu vorgesehen ist, zumindest einen Teil der elektromagnetischen Strahlung, und zwar den UV-Anteil, zu reflektieren, wobei der optische Blendschutzfilter (14) zumindest eine Antireflexionseinheit (36) aufweist, welche im getragenen Zustand einem Benutzerauge (60) zugewandten Seite des optischen Blendschutzfilters (14) angeordnet ist und wobei die Antireflexionseinheit (36) zusätzlich auf einer im getragenen Zustand dem Benutzerauge (60) abgewandten Seite des optischen Blendschutzfilters (14) angeordnet ist.

2. Schutzkassette nach Anspruch 1, **dadurch gekennzeichnet, dass** die Durchlässigkeit des optischen Blendschutzfilters (14) in zumindest einer Dunkelstufe einer Schutzstufe von zumindest 3 entspricht.

3. Schutzkassette nach Anspruch 1 oder 2, **dadurch gekennzeichnet, dass** der optische Blendschutzfilter (14) in zumindest einer Hellstufe in zumindest einem Spektralbereich eine Transmissivität (32) von zumindest 29 % aufweist.

4. Schutzkassette nach einem der vorhergehenden Ansprüche, **dadurch gekennzeichnet, dass** der optische Blendschutzfilter (14) zumindest eine weitere Flüssigkristallzelle (20) aufweist, welche in einer Sichtrichtung (22) durch den optischen Blendschutzfilter (14) hinter und/oder vor einer Flüssigkristallzelle (16) angeordnet ist.

5. Schutzkassette nach einem der vorhergehenden Ansprüche, **dadurch gekennzeichnet, dass** die passive Filtereinheit (24) in zumindest einem Spektralbereich eine Transmissivität (32) von zumindest 80 % aufweist.

6. Schutzkassette nach einem der vorhergehenden Ansprüche, **dadurch gekennzeichnet, dass** die passive Filtereinheit (24) in zumindest einem Spektralbereich eine Transmissivität (32) von höchstens 5 % aufweist.

7. Schutzkassette nach einem der vorhergehenden Ansprüche, **dadurch gekennzeichnet, dass** das dichroitische Filterelement (26) als eine Beschichtung (54) eines zumindest teilweise transparenten Substrats ausgebildet ist, welche auf einer der Flüssigkristallzelle (16) zugewandten Seite der Abdeckscheibe (30) angeordnet ist.

8. Schutzkassette nach einem der vorhergehenden Ansprüche, **dadurch gekennzeichnet, dass** das Infrarotfilterelement (28) als zumindest ein Absorptionsglas ausgebildet ist.

9. Blendschutzvorrichtung, mit einer Schutzkassette (10) nach einem der vorhergehenden Ansprüche.

## Claims

1. Protective cartridge for a glare protection device (12),
with an optical glare protection filter (14) that comprises at least one liquid-crystal cell (16), with at least one control and/or regulation unit (18) that is configured to control and/or regulate, depending on a captured work state, on an electronic signal from an external signal source and/or on a light irradiation, a transmittance of the optical glare protection filter (14) between at least one bright level and at least one dark level,
the glare protection device (12) comprising at least one 0information interface for receiving at least one piece of information about at least one work state, and/or at least one sensor for a detection of at least one work state,
the transmittance of the optical glare protection filter (14) being at at least one bright level equivalent to a protection level smaller than 2.5,
the optical glare protection filter (14) having at least one passive filter unit (24), the passive filter unit (24) comprising at least one absorptive and/or reflective infrared filter element (28),
**characterised in that** the passive filter unit (24) comprises at least one dichroic filter element (26), the dichroic filter element (26) forming a long-pass filter, the infrared filter element (28) being implemented integrally with a cover disc (30) which is arranged on a side of the optical glare protection filter (14) that faces away from the user in a state when worn,
the dichroic filter element (26) being configured to reflect at least a portion of the electromagnetic radiation, namely the UV portion,
the optical glare protection filter (14) comprising at least one anti-reflection unit (36), which is arranged on a side of the optical glare protection filter (14) that faces toward a user's eye (60) in a state when worn, and
the anti-reflection unit (36) being additionally arranged on a side of the optical glare protection filter (14) that faces away from the user's eye (60), in a state when worn.

2. Protective cartridge according to claim 1,
**characterised in that** the transmittance of the optical glare protection filter (14) is at at least one dark level equivalent to a protection level of at least 3.

3. Protective cartridge according to claim 1 or 2,
**characterised in that** the optical glare protection filter (14) has at at least one bright level in at least one spectral range a transmissivity (32) of at least 29 %.

4. Protective cartridge according to one of the preceding claims, **characterised in that** the optical glare protection filter (14) comprises at least one further liquid-crystal cell (20), which is arranged in a view direction (22) through the optical glare protection filter (14) behind and/or in front of a liquid-crystal cell (16).

5. Protective cartridge according to one of the preceding claims, **characterised in that** the passive filter unit (24) has in at least one spectral range a transmissivity (32) of at least 80 %.

6. Protective cartridge according to one of the preceding claims, **characterised in that** the passive filter unit (24) has in at least one spectral range a transmissivity (32) of maximally 5 %.

7. Protective cartridge according to one of the preceding claims, **characterised in that** the dichroic filter element (26) is realized as a coating (54) of an at least partly transparent substrate, which is arranged on a side of the cover disc (30) that faces toward the liquid-crystal cell (16).

8. Protective cartridge according to one of the preceding claims, **characterised in that** the infrared filter element (28) is embodied as at least one absorptive glass.

9. Glare protection device with a protective cartridge (10) according to one of the preceding claims.

## Revendications

1. Cassette protective pour un dispositif anti-éblouissement (12),
avec un filtre optique anti-éblouissement (14) comprenant au moins une cellule aux cristaux liquides (16), avec au moins une unité de commande et/ou régulation (18) configurée à commander et/ou réguler - en dépendance à un état de travail saisi, à un signal électronique d'une source de signaux externe et/ou à une irradiation de lumière - une transmittance du filtre optique anti-éblouissement (14) au moins entre au moins un niveau de clarté et au moins un niveau d'obscurité,
le dispositif anti-éblouissement (12) comprenant au moins une interface d'information pour recevoir au moins une information d'au moins un état de travail et/ou au moins un capteur pour une détection d'au moins un état de travail,
la transmittance du filtre optique anti-éblouissement (14) étant à au moins un niveau de clarté équivalente à un niveau de protection inférieur à 2,5,
le filtre optique anti-éblouissement (14) comprenant au moins une unité de filtre passive (24), l'unité de filtre passive (24) comprenant au moins un élément de filtre infrarouge (28) absorptif et/ou réflectif,
**caractérisée en ce que** l'unité de filtre passive (24) comprend au moins un élément de filtre dichroïque (26),
l'élément de filtre dichroïque (26) formant un filtre passe-long,
l'élément de filtre infrarouge (28) étant réalisé intégralement avec un disque couvrant (30) disposé sur un côté du filtre optique anti-éblouissement (14) détourné de l'utilisateur en état porté,
l'élément de filtre dichroïque (26) étant configuré à réfléchir au moins une partie du rayonnement électromagnétique, à savoir la partie UV,
le filtre optique anti-éblouissement (14) comprenant au moins une unité anti-reflet (36) qui est en état porté disposée sur un côté du filtre optique anti-éblouissement (14) tourné vers un œil d'utilisateur (60),
l'unité anti-reflet (36) étant disposée aussi sur un côté du filtre optique anti-éblouissement (14) détourné de l'œil d'utilisateur (60) en état porté.

2. Cassette protective selon la revendication 1,
**caractérisée en ce que** la transmittance du filtre optique anti-éblouissement (14) est à au moins un niveau d'obscurité équivalent à un niveau de protection d'au moins 3.

3. Cassette protective selon la revendication 1 ou 2,
**caractérisée en ce que** le filtre optique anti-éblouissement (14) présente à au moins un niveau de clarté dans au moins une gamme spectrale une transmissivité (32) d'au moins 29 %.

4. Cassette protective selon l'une des revendications précédentes, **caractérisée en ce que** le filtre optique anti-éblouissement (14) comprend au moins une cellule aux cristaux liquides de plus (20), qui est disposée - en direction de vue (22) à travers le filtre optique anti-éblouissement (14) - derrière et/ou avant une cellule aux cristaux liquides (16).

5. Cassette protective selon l'une des revendications précédentes, **caractérisée en ce que** l'unité de filtre passive (24) présente dans au moins une gamme spectrale une transmissivité (32) d'au moins 80 %.

6. Cassette protective selon l'une des revendications précédentes, **caractérisée en ce que** l'unité de filtre passive (24) présente dans au moins une gamme spectrale une transmissivité (32) de maximalement 5 %.

7. Cassette protective selon l'une des revendications précédentes, **caractérisée en ce que** l'élément de filtre dichroïque (26) est réalisé comme revêtement (54) d'un substrat au moins partiellement transparent, qui est disposé sur un côté de la disque couvrant (30) qui est tourné vers la cellule aux cristaux liquides (16).

8. Cassette protective selon l'une des revendications précédentes, **caractérisée en ce que** l'élément de filtre infrarouge (28) est réalisé comme au moins un verre absorptif.

9. Dispositif anti-éblouissement avec une cassette protective (10) selon l'une des revendications précédentes.
